# EUROPEAN PATENT APPLICATION

(11) **EP 3 171 302 A1**
(43) Date of publication of application: **24.05.2017**
(21) Application number: 15195142.3
(22) Date of filing: 18.11.2015
(51) Int. Cl.: G06Q 10/06, G06Q 10/10, G06Q 50/22

(54) **A METHOD FOR GENERATING AN ENTRY FOR AN ELECTRONIC LABORATORY JOURNAL**

(71) Applicant: F. Hoffmann-La Roche AG, 4070 Basel (CH); Roche Diagnostics GmbH, 68305 Mannheim (DE)
(72) Inventor: Jost, Friedrich, 6300 Zug (CH); Paul, Hubert, 82407 Wielenbach (DE); Schaper, Stefan, 82327 Tutzing (DE); Suter, Urs, 8049 Zürich (CH); Sandoz, Roger, 6343 Rotkreuz (CH); von Allmen, Bernhard, 5707 Seengen (CH)
(74) Representative: Peterreins Schley

(57) **Abstract**

In one aspect of the present disclosure a method for generating an entry for an electronic laboratory journal includes selecting a laboratory workflow specifying at least one action an operator has to carry out, wherein the action involves one or more pieces of laboratory equipment, recording one or more images or videos of the action by the operator by using a camera associated with an augmented-reality device worn by the operator and generating an entry for an electronic laboratory journal based on the recorded one or more images or videos.

## Description

### Technical Field

This disclosure relates to methods and systems for generating an entry for an electronic laboratory journal.

### Background

Today's laboratory work involves a substantial amount of documentation effort. Frequently, laboratory environments include a considerable number of complex pieces of laboratory equipment an operator has to interact with while stepping through a laboratory workflow. Moreover, the laboratory workflow itself can be fairly complex. Therefore, the required or desired amount of documentation can not only be fairly time-consuming but also lead to errors both in documentation itself (which can have serious consequences later on) as well as in the laboratory workflow (as the operator might be distracted from the actual laboratory workflow by the documentation tasks).

### Summary

This disclosure relates to methods and systems for generating an entry for an electronic laboratory journal.

In a first general aspect a method for generating an entry for an electronic laboratory journal includes selecting a laboratory workflow specifying at least one action an operator has to carry out, wherein the action involves one or more pieces of laboratory equipment, recording one or more images or videos of the action by the operator by using a camera associated with an augmented-reality device worn by the operator and generating an entry for an electronic laboratory journal based on the recorded one or more images or videos.

In a second general aspect a computer readable medium stores instructions thereon which when executed by one or more processors cause the processors to perform the operations of selecting a laboratory workflow specifying at least one action an operator has to carry out, wherein the action involves one or more pieces of laboratory equipment, recording one or more images or videos of the action by the operator by using a camera associated with an augmented-reality device worn by the operator and generating an entry for an electronic laboratory journal based on the recorded one or more images or videos.

In a third general aspect, a laboratory system includes one or more pieces of laboratory equipment, an augmented reality device, a computer readable medium storing instructions thereon which when executed by one or more processors cause the processors to perform the operations of selecting a laboratory workflow specifying at least one action an operator has to carry out, wherein the action involves one or more pieces of laboratory equipment, recording one or more images or videos of the action by the operator by using a camera associated with an augmented-reality device worn by the operator and generating an entry for an electronic laboratory journal based on the recorded one or more images or videos and one or more processors to execute the operations.

Particular embodiments of the subject matter of the first to third general aspects can be implemented so as to realize one or more of the following advantages.

Firstly, a user might more efficiently perform laboratory tasks involving data input in an electronic laboratory journal. In particular, retrieving data (e.g., image data) and including it into an electronic laboratory journal can be rendered more time efficient in some examples by exploiting the capabilities of augmented reality devices according to the present disclosure. The same can be the case for documentation tasks which involve interaction with technical laboratory equipment (e.g., analyzers) in some examples.

Secondly, a data input in an electronic laboratory journal can be rendered more reliable and less error-prone in some examples. For instance, image recognition techniques can be used to automatically register certain actions in the course of a laboratory workflow. In addition or alternatively, documentation standards can be enforced in a more efficient manner in some examples. For instance, image recognition methods can be used to evaluate whether an image of a sample recorded by the augmented reality device is sufficiently sharp.

Thirdly, particular documentation tasks relating to keeping an electronic laboratory journal can be carried out in a simplified manner. For instance, particular documentation tasks can be triggered in a hands-free manner (e.g., by voice command or gaze command). This can make the laboratory workflow faster. In addition, a hands-free operation can be advantageous in certain laboratory environments where high cleanliness requirements are set (e.g., a clean room). In some examples, interactions which other input devices used in some known documentation systems (e.g., keyboards) can be avoided.

Fourthly, distraction of an operator walking through a laboratory workflow by the documentation requirements can be reduced or avoided in some examples. In this manner, the operator can concentrate on the actual laboratory workflow which might reduce a number of errors.

A number of terms are used in the present disclosure in a particular way:
The term 'piece of laboratory equipment' as used herein can refer to any kind of automated or semi-automated or manually-operated technical device for use in laboratory work in the clinical, chemical, biological, immunology or pharmaceutical area or the like. Such a laboratory device may comprise, amongst others, at least one of a pipettor, a stirrer, a tempering device, a shaker, or an agitator. In other examples, a piece of laboratory equipment can include an analysis system or a work-cell of an analysis system or analyzer. For example, a piece of laboratory equipment can be an analyzer for analyzing a mechanical, optical, chemical or biological property of a sample.

The term 'piece of laboratory equipment' also includes sample holders for holding biological samples or reagents processed in the laboratory workflow. For instance, a sample holder can include a vessel (e.g., a test tube) or a slide for supporting a sample.

In still other examples, a 'piece of a laboratory equipment' can include disposables handled or used in laboratory work in the clinical, chemical, biological, immunology or pharmaceutical area. For instance, disposables can be syringe tips, pipette tips, vessels or slips for holding samples or reagents or other disposables.

In still other examples, a 'piece of laboratory equipment' can include a container holding reagents to be used in a laboratory workflow. For example, a container holding reagents can be a cassette for an analyzer including one or more reagents.

Accordingly, an 'action an operator has to carry out involving one or more pieces of laboratory equipment' can include handling, using, storing or operating the above discussed pieces of laboratory equipment.

For instance, an action an operator has to carry out can include preparing a biological sample or a reagents for an analysis process. This can involve, e.g., transferring a biological sample (or a portion of a sample) between two vessels, sampling a portion of a biological sample, mixing a biological sample with other substances (e.g., reagents), heating a sample, stirring a sample or filtrating a sample.

In other examples, and action the operator has to carry out can include performing an analysis step on a biological sample. This can involve operating an automated, semi-automated or manually-operated analyzer (e.g., storing one or more samples in the analyzer, setting operation parameters of the analyzer or checking and documenting measurement results obtained by the analyzer).

In still other examples, an action the operator has to carry out can include checking a status or an attribute of a piece of laboratory equipment or a sample to be processed in a laboratory workflow. For instance, an operator might have to check if a piece of laboratory equipment is clean or if a sample is clotted as part of a particular laboratory workflow.

The term 'workflow' as used herein encompasses any task that comprises a number of steps, such as for maintenance or operation of the system or one of its system components.

The term 'step of a workflow' or 'task of a workflow' as used herein encompasses any activity belonging to a workflow. The activity can be of an elementary or complex nature and is typically performed at or by means of a single piece of laboratory equipment.

A 'piece of laboratory' not necessarily is located in a dedicated laboratory. Rather, the term also includes stand-alone pieces of laboratory equipment for carrying out analytic procedures in the clinical, chemical, biological, immunology or pharmaceutical area. For example, a benchtop device in point-of-care settings such as physician clinics or pharmacies or a device for home-use can also be a piece of laboratory equipment according to the present disclosure. An 'analysis system' as used herein comprises a control unit operatively coupled to one or more analytical; pre- and post-analytical work cells wherein the control unit is operable to control the work cells. In addition, the control unit may be operable to evaluate and/or process gathered analysis data, to control the loading, storing and/or unloading of samples to and/or from any one of the analyzers, to initialize an analysis or hardware or software operations of the analysis system used for preparing the samples, sample tubes or reagents for said analysis and the like.

The term 'analyzer' / 'analytical work cell' as used herein encompasses any apparatus or apparatus component that can induce a reaction of a biological sample with a reagent for obtaining a measurement value. An analyzer is operable to determine via various chemical, biological, physical, optical or other technical procedures a parameter value of the sample or a component thereof. An analyzer may be operable to measure said parameter of the sample or of at least one analyte and return the obtained measurement value. The list of possible analysis results returned by the analyzer comprises, without limitation, concentrations of the analyte in the sample, a digital (yes or no) result indicating the existence of the analyte in the sample (corresponding to a concentration above the detection level), optical parameters, DNA or RNA sequences, data obtained from mass spectroscopy of proteins or metabolites and physical or chemical parameters of various types. An analytical work cell may comprise units assisting with the pipetting, dosing, and mixing of samples and/or reagents. The analyzer may comprise a reagent holding unit for holding reagents to perform the assays. Reagents may be arranged for example in the form of containers or cassettes containing individual reagents or group of reagents, placed in appropriate receptacles or positions within a storage compartment or conveyor. It may comprise a consumable feeding unit. The analyzer may comprise a process and detection system whose workflow is optimized for certain types of analysis. Examples of such analyzer are clinical chemistry analyzers, coagulation chemistry analyzers, immunochemistry analyzers, urine analyzers, nucleic acid analyzers, used to detect the result of chemical or biological reactions or to monitor the progress of chemical or biological reactions.

The term 'communication network' as used herein encompasses any type of wireless network, such as a WIFI, GSM, UMTS or other wireless digital network or a cable based network, such as Ethernet or the like. In particular, the communication network can implement the Internet protocol (IP). For example, the communication network comprises a combination of cable-based and wireless networks. The augmented reality devices discussed in the present disclosure can be adapted to communicate with a laboratory management system (e.g., a laboratory management system managing an electronic laboratory journal) and/or with a piece of laboratory equipment having appropriate communication capabilities through a communication network.

A 'control unit' controls an automated or semi-automated piece of laboratory equipment in a way that the necessary steps for the processing protocols are conducted by the automated piece of laboratory equipment. That means the control unit may, for example, instruct the automated piece of laboratory equipment to conduct certain pipetting steps to mix the liquid biological sample with reagents, or the control unit controls the automated system to incubate the sample mixtures for a certain time etc. The control unit may receive information from a data management unit regarding which steps need to be performed with a certain sample. In some embodiments, the control unit might be integral with the data management unit or may be embodied by a common hardware. The control unit may, for instance, be embodied as a programmable logic controller running a computer-readable program provided with instructions to perform operations in accordance with a process operation plan. The control unit may be set up to control, for example, any one or more of the following operations: loading and/or wasting and/or washing of cuvettes and/or pipette tips, moving and/or opening of sample tubes and reagent cassettes, pipetting of samples and/or reagents, mixing of samples and/or reagents, washing pipetting needles or tips, washing mixing paddles, controlling of a light source, e.g. selection of the wavelength, or the like. In particular, the control unit may include a scheduler, for executing a sequence of steps within a predefined cycle time. The control unit may further determine the order of samples to be processed according to the assay type, urgency, and the like. The augmented reality devices of the present disclosure can be configured to communicate with a control unit of an automated or semi-automated piece of laboratory equipment to retrieve information regarding a laboratory workflow for storage in an electronic laboratory journal.

### Description of the Drawings

**FIGS. 1-2** illustrate a flow chart an example method for generating an entry for an electronic laboratory journal according to the present disclosure.
**FIG. 3** is a schematic illustration of a laboratory workflow during which electronic laboratory journal entries are generated by using an augmented reality device according to the present disclosure.
**FIG. 4** illustrates an example field of view of an augmented reality device according to the present disclosure.

### Detailed Description

The methods and systems for generating an entry for an electronic laboratory journal by using an augmented reality device will subsequently be discussed in more detail.

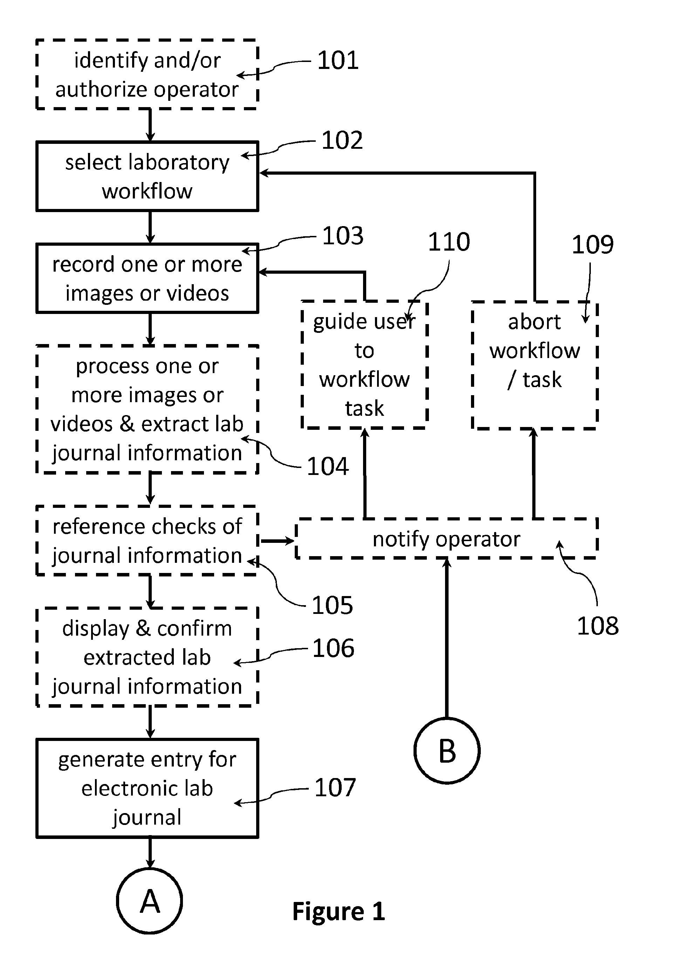

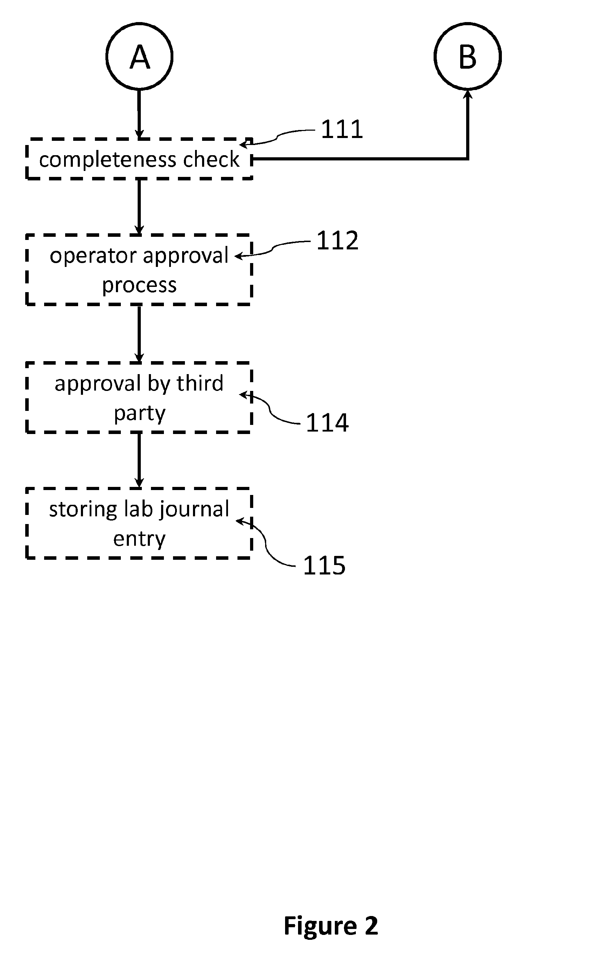

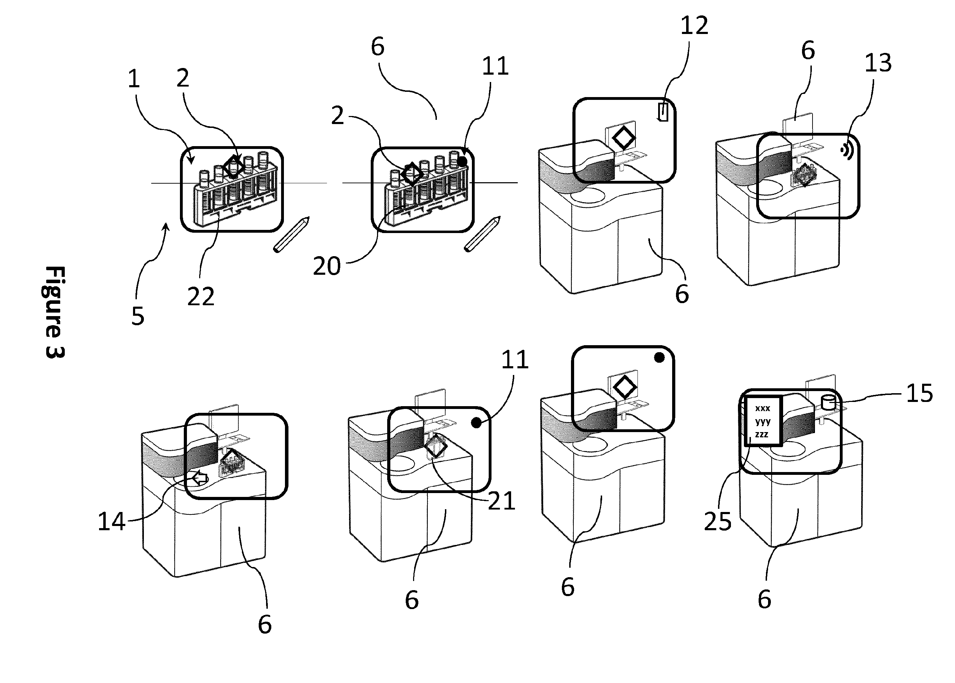

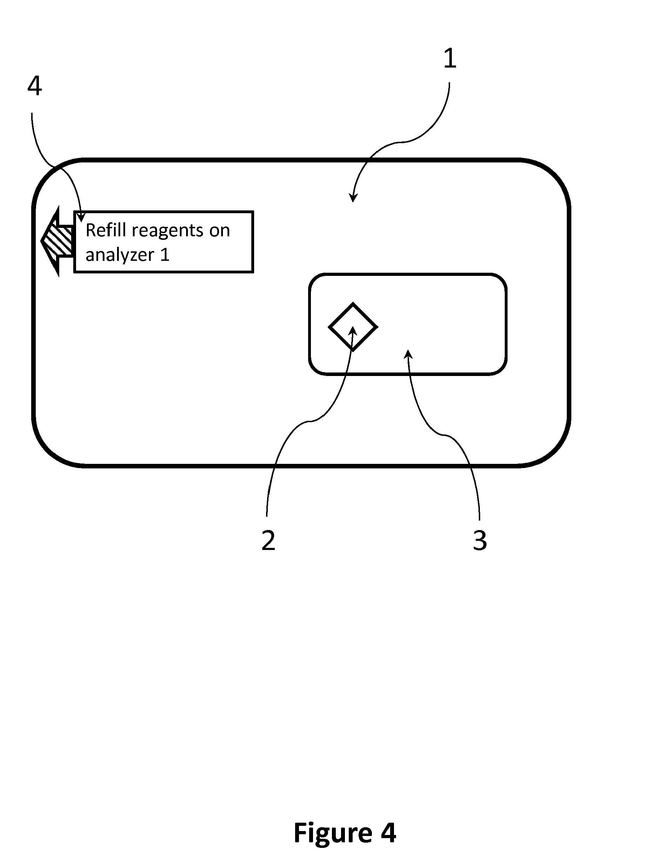

First, different steps of methods and features of systems for generating an entry for an electronic laboratory journal will be discussed in connection with **FIG. 1** and **FIG. 2**. Subsequently, additional aspects of the systems for generating an entry for an electronic laboratory journal of the present disclosure will be explained in connection with **FIG. 3**. Last, aspects of augmented reality devices that can be used in the systems and methods of the present disclosure will be detailed in connection with **FIG. 4**.

**FIGS. 1-2** illustrate a flow chart an example method for generating an entry for an electronic laboratory journal according to the present disclosure including selecting 101 a laboratory workflow specifying at least one action an operator has to carry out, the action involving one or more pieces of laboratory equipment, recording 102 one or more images or videos of the action by the operator by using a camera associated with an augmented-reality device worn by the operator and generating 107 an entry for an electronic laboratory journal based on the recorded one or more images or videos.

Different features of these steps and additional method steps according to the present disclosure will be subsequently discussed in more detail. The operations will be discussed in a particular sequence. However, in other examples of the methods of the present disclosure this sequence can be different (unless a particular operation necessarily has to be carried out prior to a second operation). In addition, the method according to the present disclosure can include only a selection of these method steps.

At step 101, an operator can be identified, authorized, or both. In general, authorization of a user can include determining an identity of the operator and determining if the operator is authorized to carry out particular workflow tasks or not based on a determined identity.

This operation can include one or more of the steps of receiving biometric data from the operator, communicating with a security token carried by the operator, pairing a device associated with the operator with another device and receiving a user ID or password. The biometric data may include one or more of a fingerprint, a retinal scan, a voice sample or a face scan, or a scan of another unique feature of the operator, or any data retrieved from processing these data items.

In some examples, the augmented reality device can be configured to record data for identifying or authorizing an operator (e.g., biometric data of the operator). However, in other examples, a different device can be configured to record data for identifying or authorizing an operator. For example, a stand-alone identification terminal can be provided in a laboratory space, or a particular piece of laboratory equipment (e.g., an analysis system or an analyzer work-cell) can be equipped with an identification terminal.

For instance, the augmented reality device can be adapted to carry out a retinal scan, a fingerprint scan or a voice recognition operation to identify an operator. In addition or alternatively, the augmented reality device can communicate with a security token carried by the operator (e.g., an identification tag) to identify or authorize the operator. In still other examples, the augmented reality device might itself be configured to identify the operator wearing the particular augmented reality device (e.g., by a unique ID of the augmented reality device communicated to a laboratory management system or a piece of laboratory equipment).

After the information regarding the operator's identity has been determined in one or more ways as described above, this information can subsequently be processed by a laboratory management system to determine if the user is authorized to conduct a particular laboratory workflow and/or to select a number of laboratory workflows the operator is authorized to conduct.

In addition or alternatively, an identity of the operator can be stored in the entry for the electronic laboratory journal.

At step 102, a laboratory workflow can be selected. In one example, the laboratory workflow is selected by a voice command, a gesture command, a gaze command or a haptic command, by monitoring the behavior of the operator or by recognizing a predetermined visual cue in an image recorded by the camera.

For example, the operator might move towards a particular piece of laboratory equipment. This can by registered by a camera. In other examples, a sensor can detect a proximity of the operator to a particular piece of laboratory equipment. In still other examples, the operator might direct his or her gaze in a direction of a particular piece of laboratory equipment.

In one example, the commands to select a particular workflow can be registered by the augmented reality device (e.g., a camera integrated into the augmented reality device). For instance, the augmented reality device can be equipped with a gaze direction detector. In this case, the augmented reality device (or a laboratory management system processing data of the augmented reality device) can be configured to select one or more particular workflows based on a gaze direction of the operator. This may include evaluating a direction and/or a duration of the gaze of the operator.

For instance, a particular workflow can include using a particular piece of laboratory equipment. Then, when it is detected that an operator looks at this particular piece of laboratory equipment for a predetermined duration, the particular workflow involving this piece of laboratory equipment can be selected. In other examples, an operator might start to handle a particular sample holder (e.g., a particular type of test tube). The laboratory workflow can be selected based on determining the type of identity of the particular sample holder.

In still other examples, a camera signal of a camera integrated into the augmented reality device can be processed to select a laboratory workflow. For example, a piece of laboratory equipment can be identified (e.g., by employing automated image recognition techniques) in a camera signal of the integrated camera. In addition, a position and/or a movement direction of the operator can be determined from the camera signal of a camera of the augmented reality device. A particular laboratory workflow can be selected based on the position and/or movement direction of the user. The camera signal of other cameras than a camera of the augmented reality device can be used in other examples.

In other examples, one or more pieces of laboratory equipment can register the commands to select a particular workflow. In still other examples, a camera installed in the laboratory space can register the command to select a particular workflow.

For instance, the operator can approach an analyzer (or other piece of laboratory equipment) and begin to interact with the analyzer (e.g., type in a password to activate the analyzer). This interaction can be registered by the analyzer and processed by the laboratory management system to select a particular workflow.

In other examples, the techniques discussed above can be used to compile a selection of one or more workflows involving the piece of laboratory equipment identified as discussed above. The compilation of laboratory workflows can be presented to the operator for selecting a particular laboratory (e.g., on a display of the augmented reality device). This selection process can be triggered by a voice command, a gesture command or a gaze command of the operator.

In some of the examples discussed above, a workflow can be selected in a seamless manner (e.g., by evaluating an operator's behavior). In this manner, the operator can concentrate on doing his or her actual tasks in the laboratory with fewer distractions due to interaction with the laboratory management system.

After a particular laboratory workflow has been selected in step 102, the operator can start to walk through different tasks of the selected laboratory workflow. Information regarding a current laboratory workflow and/or current task or action can be displayed on a display of the augmented reality device.

At some point during conducting a task of the laboratory workflow, one or more images or videos are recorded at step 103.

For instance, recording the one or more images or videos can be triggered by a voice command, a gesture command, a gaze command or a haptic command, or a combination of two or more of these commands.

In one example, the augmented reality device can include a haptic sensor an operator may interact with to trigger the recording of an image or video. For instance, the Google Glass™ device includes a touch pad at a frame of the device which can be used to trigger recording of an image or video. In other examples, the augmented reality device can be associated with other haptic sensors to trigger the recording.

In further examples, the augmented reality device can be adapted to register a gesture of the operator predefined as a command to register an image or a video.

In still other examples, the augmented reality device can be equipped with a microphone to register a voice command of the user.

The different commands described above can also be registered by cameras, microphones or other detectors not integrated in or associated with the augmented reality device. For instance, a central camera or microphone arranged in a particular laboratory space can register the commands.

In some of the preceding examples, an operator actively triggers the recording of the image or video. In further examples, the one or more images or videos is automatically triggered based on information associated with the laboratory workflow.

For instance, automatically triggering the recording of the one or more images or videos can happen based on a determination that conduction of a particular task of the laboratory workflow has started, is underway or has been completed. This can be determined based on a camera signal of the augmented reality device (or a different camera), or based on a signal generated by another piece of laboratory equipment.

In one example, closing or opening a door of an analyzer can be a trigger for recording an image or video. In still other examples, scanning a barcode of a patient sample to be analyzed can be a trigger for recording an image or video.

In still other examples, a camera of the augmented reality device can constantly record images or videos while a particular workflow tasks or laboratory workflow is being executed.

In a subsequent step 104, the one or more images or videos are processed and laboratory journal information is extracted from the one or more images or videos. This can involve one or more of the operations discussed subsequently.

In some examples, extracting laboratory journal information can involve performing one or more image recognition operations on the one or more images or videos. For instance, the one or more image recognition operations on the one or more images or videos may include one or more of identifying a property of the one or more pieces of laboratory equipment, identifying a property of a sample contained in the one or more pieces of laboratory equipment, identifying the one or more pieces of laboratory equipment or a part of the one or more pieces of laboratory equipment, identifying a sample contained in the one or more pieces of laboratory equipment.

Identifying a property of a piece of laboratory equipment can include determining an identifier of a piece of laboratory equipment.

In one example, the identity of a piece of laboratory equipment is determined based on a code (e.g., a barcode or a QR code) attached to the particular piece of laboratory equipment. For instance, the recorded one or more images or videos can contain a picture of the code. This picture can be processed by one or more image recognition operations to obtain an identifier of the respective piece of laboratory equipment (e.g., an ID or a serial number).

In other examples, the identity of a piece of laboratory equipment is determined based on alphanumeric information arranged at the particular piece of laboratory equipment. For instance, a particular piece of laboratory equipment can be equipped with an instrument name (e.g., "stirrer 12"). Again, one or more image recognition operations can be employed to extract this information and obtain an identifier of the respective piece of laboratory equipment.

In other examples, identifying a property of a piece of laboratory equipment can include determining a type of a piece of laboratory equipment.

For instance, a type of sample which is processed can be determined based on one or more properties of the sample holder containing the sample or the sample contained in the sample holder. These properties can include one or more of the list consisting of a shape or color of the sample holder, a shape or color of the cap of a sample holder or information included in a label of the sample or a visual appearance of the sample contained in the sample holder.

In one example, a sample tube with a green cap can mean that the sample contained in the sample holder is a particular whole blood sample intended for a particular analytical process. By using the method of the present disclosure, the green cap of the sample tube can be identified in a picture of the sample tube (e.g., when the operator debags the sample). Based on this, the type of the sample can be inferred (and a particular workflow can be selected in some examples).

In the previous example it becomes clear that an order of the steps of using an augmented reality device in a laboratory workflow can happen in a different order (or, at least partially, simultaneously) compared to the order shown in the flow chart of **FIG. 1** and **FIG. 2**.

In other examples, a type of a technical device can be determined by performing one or more image recognition operations on the one or more pictures or videos. For instance, there might exist a plurality of types of different technical devices for performing a particular task in a laboratory (e.g., types of manually operated pipettors).

Moreover, it might be possible to determine a property of the piece of laboratory equipment by performing one or more image recognition operations on the one or more images or videos.

In one example, determining a property of the piece of laboratory equipment can include analyzing the visual appearance of a sample contained in a sample holder. For instance, analyzing the visual appearance of a sample can include one or more of analyzing a color of the sample, determining if the sample has particular transmission or scattering properties and determining if the sample has a particular shape or includes features with a particular shape. These visual cues can be used to determine a property of the sample (e.g., a presence of certain interferences, an indication that the sample is corrupted, or a physical or chemical property of the sample itself like sample volume).

In one example, analyzing a color of the sample can be used to determine the presence of interferences in blood samples (e.g., lipemic, hemolytic or icteric interferences). In another example, analyzing if the sample has a particular shape or includes features with a particular shape can be used to determine that a blood sample is clotted.

In other examples, determining a property of the piece of laboratory equipment can include determining a status or operational parameters of a technical device.

For instance, an operational parameter of a technical device might be displayed on a display of the technical device. By using the method of the present disclosure, this operational parameter can be retrieved from the one or more images or videos by image recognition techniques. In other examples, an operational parameter of the technical device can be inferred from visual cues in the one or more images or videos (e.g., an operator pressing a predetermined button).

In still other examples, determining a property of the piece of laboratory equipment can include determining a position of a sample holder in another piece of laboratory equipment. For instance, in cases where multiple sample holders are handled at a time (e.g., test tubes arranged in a rack), a position of the particular sample holders can be determined by using image recognition techniques.

The extracted information discussed above can be included in an electronic laboratory journal, as will be discussed in more detail below. As already mentioned, in some other examples the recorded images or videos are not processed by image recognition techniques to extract information but simple provided for storage in the electronic laboratory journal. In other examples, the one or more images or videos are provided for storage in addition to information extracted from the one or more images by image processing techniques.

In the above examples, it has been described that information to be stored in an electronic laboratory journal can be extracted from the recorded one or more images or videos. In addition or alternatively, information to be stored in an electronic laboratory journal can also be retrieved from other sources.

In one example, a piece of laboratory equipment can transmit data to be stored in an electronic laboratory journal (e.g., over a short range communication network of the laboratory). For instance, a piece of laboratory equipment can transmit operational properties of the device (e.g., a set of operation parameters used for a particular analysis) or results of an analysis operation performed by the piece of laboratory equipment or other sensor data of the piece of laboratory equipment to be stored in an electronic laboratory journal. This data transmission process can be triggered by one or more voice commands, gaze command, gesture commands or haptic commands of the operator (which can be registered by the augmented reality device in some examples). In other examples, this data transmission process can be triggered by the augmented reality device (e.g., by using an RFID tag or a proximity sensor of the piece of laboratory equipment and/or the augmented reality device).

In a further step 105, the entry for an electronic laboratory journal can be compared with one or more reference values.

For instance, a comparison with one or more reference values can include determining if the entry for an electronic laboratory journal meets one or more predefined requirements for an entry documenting a particular task of the selected laboratory workflow.

In one example, the entry for a particular task might require particular data (e.g., a picture of a sample or a piece of laboratory equipment). At step 105, it can be determined if this data has been retrieved. In addition or alternatively, the comparison with one or more reference values can include determining if one or more image parameters of the one or more images or videos meet predetermined requirements. For example, it can be determined if the one or more images meet a sharpness or illumination requirement. In addition or alternatively, the comparison with one or more reference values can include determining is one or more expected objects are present in the one or more images or videos.

In other examples, comparison with one or more reference values can include processing one or more of the properties of the pieces of laboratory devices or samples described in connection with step 104. For instance, it can be determined if a sample volume is below a predetermined threshold volume or if the sample includes a particular interference. Depending on a result of the reference check at step 105, the method can proceed to step 108 if the reference check has not been passed or to step 106 if the reference check has been passed.

At step 108, an operator is notified that a reference check has not been passed. In one example, this can include displaying a notification information on a display of the augmented reality device worn by the operator.

Depending on a type of error the process can then continue either with step 110 when particular data is missing or with step 109 and abort a particular laboratory workflow or workflow task.

In one example of the former case, a specified piece of data might be missing in the entry for the electronic laboratory journal. For instance, a particular image or video of a piece of laboratory equipment can be missing. The operator can be informed regarding this missing piece of data.

In other examples, a sample property extracted from the one or more images or videos might indicate that a sample is spoiled by interferences in which case the operator can be notified and the laboratory workflow can be aborted.

In the case when the reference checks have been passed the extracted laboratory journal information can be displayed to the operator. This can happen on a display of the augmented reality device or on a different display (e.g., a workstation of the laboratory). The operator can confirm the displayed laboratory journal information in an additional step.

In a subsequent step, the method proceeds to step 107 including generating an entry for the electronic laboratory journal once all information for a laboratory journal entry regarding a particular laboratory workflow or workflow task has been collected.

The information can be organized in a predetermined data structure comprising one or more of a device identifier of a piece of laboratory equipment involved in the laboratory workflow, a time stamp including timing information regarding the performed laboratory workflow task, identifying information regarding the operator having performed the laboratory workflow task, the one or more images or videos recorded an information extracted from the one or more images or videos recorded.

At step 111, the generated entry for an electronic laboratory journal can be checked for completeness. This completeness check can (similar to the reference check at step 105) include determining if a piece of required data is missing in the entry for the electronic laboratory journal. If the check is not passed, a user can be notified in a similar manner as discussed above in connection with step 105.

At step 112, the operator can approve the electronic laboratory entry. This can involve digitally signing the entry for the electronic laboratory journal. In one example, approving the electronic laboratory entry includes signing the entry for the electronic laboratory journal by an electronic signature of the operator in response to one or more voice commands, gaze command, gesture commands or haptic commands of the operator. As described above, the one or more voice commands, gaze command, gesture commands or haptic commands of the operator can be registered by a sensor (e.g., a camera) arranged at the augmented reality device.

Subsequently, at step 114, a third party can approve the electronic laboratory entry. This can involve digitally signing the entry for the electronic laboratory journal. In one example, approving the electronic laboratory entry includes signing the entry for the electronic laboratory journal by an electronic signature of the operator in response to one or more voice commands, gaze command, gesture commands or haptic commands. In one example, the third party can be a laboratory manager.

Eventually, at step 115, the entry for the electronic laboratory journal is stored in the electronic laboratory journal.

As becomes clear in the above description, the methods of the present disclosure can employ an augmented reality device in various ways in a generation process of an entry for an electronic laboratory journal to reduce a documentation burden of an operator and/or increase a reliability of a documentation process.

An overall overview over different aspects of the techniques of the present disclosure has been given in connection with **FIG. 1** and **FIG. 2** above. Subsequently, a concrete example of a method of the present disclosure will be discussed in connection with **FIG. 3** for the sake of illustration. Additional general aspects of the techniques of the present disclosure will also be discussed in this context.

In the example of **FIG. 3**, the laboratory workflow is a workflow taking place in a laboratory environment to determine if a patient has a particular infectious disease. The workflow involves an automated analyzer 6 (e.g., a spectrophotometric analyzer for biological samples or a different type of analyzer), a rack 22 containing a plurality of biological samples to be analyzed a plurality of test tubes 20 containing the biological samples and a reagent cassette 21 including one or more reagents to be used in the analysis process(i.e., four pieces of laboratory equipment) and includes different tasks to be carried out by an operator wearing an augmented reality device. The example augmented reality device is adapted to create an overlay 1 over the operator's field of view and a focus indicator 2 for object detection. Further and alternative aspects of augmented reality devices of the present disclosure will be discussed in connection with **FIG. 4** below.

In a first step (left right image), the operator starts to handle a rack 22 containing a plurality of sample tubes including biological samples to be analyzed. The focus indicator 2 can identify a cap of one of the sample tubes contained in the rack (e.g., a color or form of the cap of the sample tube). For instance, this operation can include processing an image of the rack 22 taken by a camera of the augmented reality device. The cap of the sample tube can indicate a type of biological sample contained in the sample tube 22. In the example of **FIG. 3**, the cap might indicate that the sample is a diluted blood sample to be analyzed to determine a particular set of clinical parameters.

Based on a gaze command of the operator (e.g., the operator looking at the rack 22 or a cap of a test tube in the rack 22 for longer than a predetermined period of time), a laboratory workflow associated with the particular biological sample can be selected. As discussed above, different commands than gaze commands can be used in other examples. Furthermore, as discussed above, an identity of the operator can be determined. For example, the augmented reality device can record a voice sample of the operator and determine the operator's identify based on the voice sample (or by another identification method using biometric data discussed in the present disclosure). For instance, the operator can be prompted to identify himself or herself by a notice displayed by the augmented reality device. The identity information can be stored to be included in the electronic laboratory journal.

In a second step, the operator might check if all samples in the test tubes 20 contained in the rack 22 are in an acceptable state. The augmented reality device can record an image or a video of this process and determine, e.g., one or more parameters of the biological samples contained in the sample tubes 20. For instance, the one or more parameter can include a volume of the biological samples and a color of the biological samples. Again, the operator can trigger this recording by one or more of the commands discussed above (e.g., a gaze command). The retrieved information regarding the one or more parameters of the biological samples can be checked against a reference value to determine whether the biological samples are in an appropriate condition for the analytic test. For example, it can be checked if the color of the samples matches an expected color and/or if the volume of the sample exceeds a minimum volume required to perform a particular test. The operator can be informed if one or more samples do not pass the checks. In addition or alternatively, the retrieved one or more parameters can be stored for inclusion into an electronic laboratory journal.

As can be seen in **FIG. 3**, the augmented reality device might display indicators 11, 12, 13, 14, 15 of a current operation in the overlay 1 of the operator's field of view. For instance, recording of an image or video is indicated by a particular indicator 11, whereas communication with a device is indicated by a second indicator 13.

In a subsequent step (third picture in the upper row), the operator starts and programs the automated analyzer 6. During this process, operational parameters of the automated analyzer 6 for storage into an electronic laboratory journal can be retrieved from an image or video recorded by the augmented reality device.

In a further step, the operator scans a barcode on the sample tubes 20 included in the rack 22 containing to determine an identity of the respective sample (e.g., information identifying a patient) and an arrangement of the respective samples in the rack 22. In the example of **FIG. 3**, an image or video taken by the augmented reality device is processed to retrieve this information. In addition, the augmented reality device can communicate with the automated analyzer 6 and provides the sample identity information and arrangement information to the automated analyzer. Moreover, this information can be provided for inclusion into an electronic laboratory journal.

In a subsequent step (first picture in the lower row of **FIG. 3**), the operator is about to start loading the sample tubes contained in the rack into the automated analyzer 6. The augmented reality device can display information regarding this task in a respective display indicator 14. This can help the operator to either identify a subsequent step of the laboratory workflow or properly carry out the respective step (e.g., by displaying helpful information regarding the workflow task).

In a further step (second picture in the lower row), the operator starts to introduce a reagent cassette 21 containing one or more reagents to be used in the analysis process into the automated analyzer 6. Again, this process can be recorded by the augmented reality device (e.g., triggered by a gaze command of the operator). In a similar manner as discussed above for the samples, the recorded image or video ca be processed to determine a type or an identity of the reagents included in the reagent cassette or one or more parameters of the reagents. Moreover, one or more reference checks can be performed on the retrieved type or identity of the reagents included in the reagent cassette or the one or more parameters of the reagents. For instance, it can be checked if the reagents are the appropriate reagents for the selected laboratory workflow. In addition or alternatively, it can be checked if an expiry date of the reagents has passed. Again, the operator can be notified regarding the results of the one or more reference checks. In addition or alternatively, the retrieved information regarding the reagents can be processed for storage in the electronic laboratory entry.

In a penultimate step (third picture in the lower row), a user starts the automated analysis process. The augmented reality device can record this process in an image or video (e.g., triggered by a gesture command of the operator 5 pressing a button of the automated analyzer 6). This image or video can be processed to retrieve operational parameters of the automated analyzer 6 or results of the analysis process by the automated analyzer 6 for storage in an electronic laboratory journal. Moreover, the image or video can be processed to record error messages of the automated analysis device 6.

In a final step (last picture in the bottom row), the augmented reality device can display information regarding the electronic laboratory entry 25 in the field of view of the operator 5. For example, the information can include a summary of the information collected in the previous steps. The operator can check this information 25 and can authorize storage of the electronic laboratory entry by a voice command (or by another command discussed in the present disclosure). Then, an entry for an electronic laboratory entry is generated and stored. After several aspects of the methods to generate an entry of an electronic laboratory journal have been discussed in connection with **FIG. 1**, **FIG. 2** and **FIG. 3** additional aspects of augmented reality devices to be used for the techniques of the present disclosure will be subsequently discussed in connection with **FIG. 4**.

In general, the augmented reality device employed in the techniques of the present disclosure can include a head-up display, a head-mounted display, eyeglasses including a display, contact lenses including a display and a virtual retina display for displaying the information discussed above.

In addition or alternatively, one or more cameras configured to record images and videos can be integrated into the augmented reality device. For example, a camera can be arranged at the augmented reality device pointing substantially in a gazing direction of the operator.

In addition or alternatively, the augmented reality device can include an input interface for retrieving commands of the operator (e.g., to trigger any operation described above). The interface can be configured to retrieve one or more of a voice command, a gesture command, a gaze command or a haptic command.

In one example, the augmented reality device can include a microphone to retrieve voice commands of the operator. In another example, the augmented reality device can include a touchpad to retrieve a haptic command of the operator (e.g., a touchpad as provided in the Google Glass™ augmented reality device). In still other examples, the augmented reality device includes a gaze direction determination device for retrieving gaze commands. For example, a gaze direction determination device can include sensors determining a gaze direction of the operator directly or indirectly (e.g., by analyzing a heading or orientation of the augmented reality device).

In still other examples, the augmented reality device can include a camera to retrieve gesture commands of the operator. The camera can be the same camera adapted to record the one or more images or videos of the pieces of laboratory equipment in the techniques of the present disclosure.

In the previous sections, it has been described that the augmented reality device is equipped with different interfaces (which might therefore move with the operator). However, in other examples commands of the operator can be retrieved by interfaces of other device connected with the augmented reality device in a communication network. For instance, a camera or microphone of a piece of laboratory equipment or installed in a laboratory room can be configured to retrieve commands of the operator.

In the same manner, processing of commands or of any other information (e.g., image or video data) described in the present disclosure not necessarily takes place locally (e.g., at the augmented reality device or on a particular piece of laboratory equipment). Rather, the processing of commands and any other information described in the present disclosure can take place at any computer system of a laboratory network in communication with the augmented reality device (and in communication with pieces of laboratory equipment having communication capabilities over a communication network in some examples).

In addition or alternatively, processing of commands or of any other information (e.g., image or video data) described in the present disclosure can also take place at a remote server or in the cloud.

For instance, image recognition operations as described herein can require considerable computational resources. These resources can be provided by a remote server or the cloud. Additionally or alternatively, the electronic laboratory journals described herein can be compiled and/or stored on a remote server or in the cloud.

Coming back to **FIG. 4**, an example field of view of an augmented reality device for performing the techniques of the present disclosure can be seen.

The example augmented reality device is adapted to overly a field of view of an operator with an overlay 1, a focus indicator 2, an indicator frame 3 for visual confirmation of an identification of an object and a task or activity indictor 4.

The overlay can delimit a portion of the operator's field of view into which the augmented reality device can project information.

As discussed above in connection with **FIG. 3**, the focus indicator 2 can indicate a gaze direction of the operator or a heading direction of the augmented reality device. For instance, a gaze direction of the operator can be determined by a gaze direction determination device and a corresponding focus indicator 2 can be displayed. In other examples, the augmented reality device can evaluate heading information of the augmented reality device and display a corresponding focus indicator 2 based on the heading information (e.g., in a similar way as in a viewfinder of an autofocus camera).

The indicator frame 3 can signal to a user that a particular object (e.g., a piece of laboratory equipment) has been selected (e.g., in response to a voice command, a gesture command, a gaze command or a haptic command, or a combination of two or more of these commands of the operator). In this manner, an operator can receive visual feedback if his or her command has been duly processed. In other examples, different visual indicators can be used to give visual feedback regarding an operator command. For instance, an indicator symbol or alphanumeric text can be displayed in the overlay 1 of the augmented reality device.

The task or activity indictor 4 can provide the operator with various information regarding the laboratory workflow and/or the process of generating an electronic laboratory journal entry. As can be seen in **FIG. 4**, the task or activity indictor 4 can indicate a next task to the operator. In other examples, the task or activity indictor 4 can give visual feedback regarding a current task of the laboratory workflow.

In some examples, the task or activity indictor 4 can give visual feedback regarding a current step of the process of generating an entry of an electronic laboratory journal (e.g., as shown in the display indicators 11, 12, 13, 14, 15 of **FIG. 3**). For instance, the task or activity indictor 4 can include information extracted from the one or more images or videos recorded in the process of generating an electronic laboratory journal.

In the example of **FIG. 4**, the augmented reality device generates an overlay covering a substantial portion of an operator's field of view. In other examples, an overlay of an augmented reality device can be configured to cover only a smaller fraction of an operator's field of view. For instance, an overlay of the augmented reality deice can be arranged in the upper left or upper right region of an operator's field of view.

In other example, the augmented reality device can be configured to display not only an overlay over an operator's field of view but also a video stream of the field of view of the operator.

In the preceding detailed description multiple examples of methods and systems for generating an entry for an electronic laboratory journal of the present disclosure have been discussed. However, the methods and systems for generating an entry for an electronic laboratory journal of the present disclosure can also be configured as set out in the following aspects:
1. A method for generating an entry for an electronic laboratory journal, the method comprising:
   selecting a laboratory workflow specifying at least one action an operator has to carry out, wherein the action involves one or more pieces of laboratory equipment;
   recording one or more images or videos of the action by the operator by using a camera associated with an augmented-reality device worn by the operator;
   generating an entry for an electronic laboratory journal based on the recorded one or more images or videos.
2. The method of aspect 1 wherein the recording is triggered by a voice command, a gesture command, a gaze command or a haptic command, or a combination of two or more of these commands.
3. The method of aspect 2 wherein the voice command, gaze command, gesture command, haptic command, or combination of two or more of these commands are registered by the augmented-reality device.
4. The method of any one of the preceding aspects wherein the camera is integrated into the augmented-reality device.
5. The method of aspect 4 wherein the camera is arranged on the augmented reality device pointing substantially in a gazing direction of the operator.
6. The method of any one of the preceding aspects wherein the augmented reality device includes one or more of a head-up display, a head-mounted display, eyeglasses including a display, contact lenses including a display and a virtual retina display.
7. The method of any one of the preceding aspects wherein the augmented reality device is configured for hands-free operation.
8. The method of any one of the preceding aspects further comprising:
   receiving data from at least one of the one or more pieces of laboratory equipment associated with the at least one action the operator carries out; and
   generating an entry for an electronic laboratory journal includes using the data received from the at least one of the one or more pieces of laboratory equipment.
9. The method of aspect 8 wherein the received data from at least one of the one or more pieces of laboratory equipment includes one or more of data regarding one or more operation parameters of the one or more pieces of laboratory equipment, sensor data of the one or more pieces of laboratory equipment or measurement data of the one or more pieces of laboratory equipment.
10. The method of aspect 9 wherein a data transmission from the at least one of the one or more pieces of laboratory equipment is triggered by the augmented reality device.
11. The method of any one of the preceding aspects wherein recording the one or more images or videos is triggered by a voice command, a gesture command, a gaze command or a haptic command, or a combination of two or more of these commands.
12. The method of any one of the preceding aspects wherein recording the one or more images or videos is automatically triggered based on information associated with the laboratory workflow.
13. The method of any one of the preceding aspects wherein recording the one or more pictures or videos is automatically triggered in response to a trigger event.
14. The method of aspect 13 wherein the trigger event is registered by the camera associated with the augmented-reality device.
15. The method of any one of the preceding aspects further comprising authentication of the operator.
16. The method of aspect 15 wherein authentication of the operator includes one or more of receiving biometric data from the operator, communicating with a security token carried by the operator, pairing a device associated with the operator with another device and receiving a user ID or password.
17. The method of aspect 16 wherein the biometric data includes one or more of a fingerprint, a retinal scan, a voice sample or a face scan, or a scan of another unique feature of the operator, or any data retrieved from processing these data items.
18. The method of aspect 16 or 17 wherein the biometric data is recorded by the augmented reality device.
19. The method of any one of aspects 15 or 18 wherein the entry in the electronic laboratory journal includes information regarding an identity of the operator.
20. The method of any one of the preceding aspects wherein the laboratory workflow is selected by a voice command, a gesture command, a gaze command or a haptic command, by monitoring the behavior of the operator or by recognizing a predetermined visual cue in an image recorded by the camera.
21. The method of aspect 20 wherein the behavior of the operator includes a movement towards a particular piece of laboratory equipment or gazing in a direction of a particular piece of laboratory equipment.
22. The method of any one of the preceding aspects wherein recording one or more images or videos of the action by the operator by a camera associated with the augmented-reality device includes recording multiple images or videos at different points in time.
23. The method of any of the preceding aspects wherein generating an entry for an electronic laboratory journal based on the recorded one or more images or videos includes performing one or more image recognition operations on the one or more pictures or videos.
24. The method of aspect 23 wherein the one or more image recognition operations on the one or more images or videos are selected based on the selected laboratory workflow.
25. The method of aspect 23 or 24 wherein the one or more image recognition operations on the one or more images or videos include one or more of identifying of a property of the one or more pieces of laboratory equipment, identifying of a property of a sample contained in the one or more pieces of laboratory equipment, identifying the one or more pieces of laboratory equipment or a part of the one or more pieces of laboratory equipment, identifying a sample contained in the one or more pieces of laboratory equipment.
26. The method of any one of the preceding aspects wherein the entry for an electronic laboratory journal includes one or more of an operator ID, an ID of the or more pieces of laboratory equipment, a time stamp, a date and a narrative of the action carried out by the operator.
27. The method of aspect 26 wherein the narrative is predefined for the laboratory workflow.
28. The method of aspect 26 or 27 wherein the narrative is generated based on voice input of the operator.
29. The method of any one of aspects 26 to 28 wherein the narrative is automatically generated in response to user interactions with the one or more pieces of laboratory equipment when carrying out the action by using a library of narrative templates.
30. The method of aspect 29 wherein a narrative template is selected based on an input received from the one or more pieces of laboratory equipment or the camera or another sensor of the augmented reality device.
31. The method of one of the preceding aspects further comprising:
   comparing the entry for an electronic laboratory journal with one or more reference values;
   if the entry for an electronic laboratory journal meets one or more criteria compared to the reference values, storing the entry in the electronic laboratory journal; and
   if the entry for an electronic laboratory journal does not meet one or more criteria compared to the reference values, notifying the operator.
32. The method of aspect 31 wherein notifying the operator includes identifying an operation in the laboratory workflow by the augmented reality device.
33. The method of one of the preceding aspects further comprising displaying at least a portion of the generated entry in the electronic laboratory journal.
34. The method of aspect 33 wherein the at least a portion of the generated entry in the electronic laboratory journal is displayed on a display of the augmented reality device.
35. The method of aspect 33 further comprising:
   receiving one or more voice commands, gaze commands, gesture commands or haptic commands after displaying at least a portion of the generated entry for the electronic laboratory journal; and
   storing the entry in the electronic laboratory journal in response to the one or more voice commands, gaze commands, gesture commands or haptic commands.
36. The method of one of the preceding aspects further comprising signing the entry for the electronic laboratory journal by an electronic signature of the operator in response to one or more voice commands, gaze commands, gesture commands or haptic commands.
37. The method of one of the preceding aspects further comprising:
   sending the entry for the electronic laboratory journal to a third party; and
   only storing the entry in the electronic laboratory journal in the laboratory journal upon approval by the third party.
38. The method of any one of the preceding aspects wherein the piece of laboratory equipment is one or more of an automated, semi-automated or manually-operated technical device, an analysis system, a work-cell of an analysis system or analyzer, a sample holder for holding biological samples or reagents processed in the laboratory workflow, a disposable handled or used in laboratory work or a container holding reagents.
39. The method of any one of the preceding aspects, further comprising generating a narrative for the entry for an electronic laboratory journal, wherein the narrative is automatically generated in response to user interactions with the one or more pieces of laboratory equipment when carrying out the action by using a library of narrative templates.
40. A computer readable medium storing instructions thereon which when executed by one or more processors cause the processors to perform the operations of the methods of any one of aspects 1 to 39.
41. A laboratory system including:
   one or more pieces of laboratory equipment;
   an augmented reality device;
   a computer readable medium storing instructions thereon which when executed by one or more processors cause the processors to perform the operations of the methods of any one of aspects 1 to 39; and
   one or more processors to execute the operations of the methods of any one of aspects 1 to 39.

## Claims

1. A method for generating an entry for an electronic laboratory journal, the method comprising:
selecting a laboratory workflow specifying at least one action an operator has to carry out, wherein the action involves one or more pieces of laboratory equipment;
recording one or more images or videos of the action by the operator by using a camera associated with an augmented-reality device worn by the operator;
generating an entry for an electronic laboratory journal based on the recorded one or more images or videos.

2. The method of claim 1 wherein the recording is triggered by a voice command, a gesture command, a gaze command or a haptic command, or a combination of two or more of these commands.

3. The method of claim 2 wherein the voice command, a gesture command, a gaze command or a haptic command, or a combination of two or more of these commands are registered by the augmented-reality device.

4. The method of any one of the preceding claims wherein the camera is integrated into the augmented-reality device.

5. The method of any one of the preceding claims wherein recording the one or more images or videos is automatically triggered based on information associated with the laboratory workflow.

6. The method of any one of the preceding claims further comprising identification or authentication of the operator, wherein authentication of the operator includes receiving biometric data from the operator by the augmented reality device.

7. The method of any one of the preceding claims wherein the laboratory workflow is selected by a voice command, a gesture command, a gaze command or a haptic command, by monitoring the behavior of the operator or by recognizing a predetermined visual cue in an image recorded by the camera, preferably wherein the behavior of the operator includes a movement towards a particular piece of laboratory equipment or gazing in a direction of a particular piece of laboratory equipment.

8. The method of any of the preceding claims wherein generating an entry for an electronic laboratory journal based on the recorded one or more images or videos includes performing one or more image recognition operations on the one or more pictures or videos.

9. The method of claim 8 wherein the one or more image recognition operations on the one or more images or videos are selected based on the selected laboratory workflow.

10. The method of claim 8 or 9 wherein the one or more image recognition operations on the one or more images or videos include one or more of identifying of a property of the one or more pieces of laboratory equipment, identifying of a property of a sample contained in the one or more pieces of laboratory equipment, identifying the one or more pieces of laboratory equipment or a part of the one or more pieces of laboratory equipment, identifying a sample contained in the one or more pieces of laboratory equipment.

11. The method of any one of the preceding claims wherein the piece of laboratory equipment is one or more of an automated, semi-automated or manually-operated technical device, an analysis system, a work-cell of an analysis system or analyzer, a sample holder for holding biological samples or reagents processed in the laboratory workflow, a disposable handled or used in laboratory work or a container holding reagents.

12. The method of any one of the preceding claims, further comprising generating a narrative for the entry for an electronic laboratory journal, wherein the narrative is automatically generated in response to user interactions with the one or more pieces of laboratory equipment when carrying out the action by using a library of narrative templates.

13. The method of one of the preceding claims further comprising:
comparing the entry for an electronic laboratory journal with one or more reference values;
if the entry for an electronic laboratory journal meets one or more criteria compared to the reference values, storing the entry in the electronic laboratory journal; and
if the entry for an electronic laboratory journal does not meet one or more criteria compared to the reference values, notifying the operator.

14. The method of one of the preceding claims further comprising signing the entry for the electronic laboratory journal by an electronic signature of the operator in response to one or more voice commands, gaze command, gesture commands or haptic commands.

15. A laboratory system including:
one or more pieces of laboratory equipment;
an augmented reality device;
a computer readable medium storing instructions thereon which when executed by one or more processors cause the processors to perform the operations of the methods of any one of claims 1 to 14; and
one or more processors to execute the operations of the methods of any one of claims 1 to 14.
